# EUROPEAN PATENT APPLICATION

(11) **EP 4 230 734 A1**
(43) Date of publication of application: **23.08.2023**
(21) Application number: 22157817.2
(22) Date of filing: 21.02.2022
(51) Int. Cl.: C12N 9/22

(54) **TYPE II CRISPR/CAS9 GENOME EDITING SYSTEM AND THE APPLICATION THEREOF**

(71) Applicant: Zhuhai Shu Tong Medical Technology Co., Ltd., Zhuhai, Guangdong 519000 (CN)
(72) Inventor: TIAN, Rui, Zhuhai, Guangdong, 519000 (CN); HUANG, Long, Zhuhai, Guangdong, 519000 (CN); XIE, Hongxian, Zhuhai, Guangdong, 519000 (CN)
(74) Representative: Meyer, Thorsten

(57) **Abstract**

The disclosure relates to a Type II CRISPR/Cas9 genome editing system, belonging to the technical field of genome editing. The genome editing system comprises a Cas9 protein, helper proteins, a CRISPR RNA and a trans-activated CRISPR RNA; wherein the Cas9 protein is a DNA endonuclease, and the Cas9 protein has an amino acid sequence as shown in SEQ ID NO: 1, or an amino acid sequence with at least 80%, 85%, 90%, 95%, 98%, or 99% homology to the amino acid sequence as shown in SEQ ID NO: 1. According to the disclosure, through bioinformatics analysis, the Type II CRSIPR/Cas9 genome editing system in the *Faecalibaculum rodentium* is discovered, and the genome editing system is applied to editing prokaryotic or eukaryotic genes and provides a new selection for a genome editing toolbox.

## Description

### TECHNICAL FIELD

The disclosure relates to a Type II CRISPR/Cas9 genome editing system derived from *Faecalibaculum rodentium,* belonging to the technical field of genome editing.

### BACKGROUND ART

Gene editing technology makes it possible to modify DNA sequence localization points. For example, the first generation of genome editing tools zinc finger nucleases, (ZFNs), the second generation of genome editing tools such as transcription activator-like effector nucleases (TALENs) can all be used to transform targeted genomes. However, these methods are difficult to design, not easy to manufacture, expensive and not universal.

The CRISPR (Clustered Regularly Interspaced Short Palindromic Repeat)/Cas (CRISPR-associated protein) system is the innate immune system derived from archaea and bacteria, serving as the third-generation genome editing tool. Different from that previous genome editing tools (protein-DNA recognition), the method utilizes the principle of nucleic acid base complementary pair to identify the target DNA sequence and guide the Cas effector protein to carry out site-specific cleaving, and has the advantages of high applicability, simple design, low cost and high efficiency. Cas protein contains a variety of different effector domains, which play roles in different activities such as nucleic acid recognition, stabilization of complex structures, and hydrolysis of DNA phosphodiester bonds. Among them, the Type II CRISPR/Cas9 system derived from streptococcus pyogene Cas (SpCas9) has become the most widely used CRISPR/Cas system due to its high cleavage efficiency. This system identifies and cleaves the Protospacer Adjacent Motif (PAM) sequence, i.e. "NGG" on the targeted polynucleotide, leaving a flat-ended overhang, and affecting genome editing.

In large and diverse metagenomes harboring uncultured or even undiscovered microorganisms, there may be a large number of undiscovered CRISPR/Cas9 systems whose activities in prokaryotes and eukaryotes, as well as *in vitro* environments, need to be confirmed.

In 2015, Dr.Byoung-Chan Kim's team isolated a new anaerobic strain ALO17 from the feces of laboratory mice C57BL/6J, and analyzed the phylogenetic relationship of the strain with the 16SrRNA gene sequence of prokaryote, and found that the strain is closely related to *Holdemanellabiformis DSM 3989T, Faecalicoccuspleomorphus ATCC 29734T, Faecalitaleacylindroides ATCC 27803T,* and *Allobaculumstercoricanis DSM 13633T* (sequence homologies are 87.4%, 87.3%, 86.9% and 86.9%, respectively). On the basis of multiple taxonomic evidences, this species is considered to be a new genus of the *Erysipelothricaceae* family, and named as *Faecalibaculum rodentium Gen.nov., sp. nov..* In the past five years, scientists in various countries around the world have carried out research on this strain in two fields, i.e., intestinal microbial environment and high-fat diet, and intestinal microbial environment and tumorigenesis. However, it has not been reported in the field of genome editing.

### SUMMARY OF THE DISCLOSURE

The purpose of the disclosure is to overcome the defects of the prior art and provide a Type II CRISPR/Cas9 genome editing system derived from *Faecalibaculum rodentium,* wherein the genome editing system has new physical and chemical properties and can identify a plurality of different PAM (protospacer adjacent motif) sequences, including NGTA and NNTA (N is A, C, G, or T).

In order to achieve the purpose, the technical solution adopted by the disclosure is as follows.

A Type II CRISPR/Cas9 genome editing system, characterized in that, the system comprises a Cas9 protein, helper proteins, a crRNA(CRISPR RNA) and a tracrRNA (trans-activated CRISPR RNA) in the functional form of an RNP complex (ribonucleoprotein complex) of the Cas9 protein and a guide RNA formed by hybridizing the crRNA with the tracrRNA; wherein the Cas9 protein is a DNA endonuclease, and the Cas9 protein has an amino acid sequence as shown in SEQ ID NO: 1, or an amino acid sequence with at least 80%, 85%, 90%, 95%, 98%, or 99% homology to the amino acid sequence as shown in SEQ ID NO: 1.

Preferably, the Type II CRISPR/Cas9 genome editing system is derived from *Faecalibaculum rodentium.*

The Cas9 protein cleaves a double-stranded DNA complementary to a crRNA upstream of a PAM sequence by a nuclease domain, wherein the nuclease domain is selected from a HNH-like nuclease domain, a RuvC-like nuclease domain, or a combination thereof.

In some embodiments, the term "ribonucleoprotein complex" is preferably referred to the "FrCas9 protein complex" according to the disclosure.

The mutations at several key amino acid sites of the specific Cas9 protein are explained in details as follows. The mutation of E to A at 796 position amino acid will result in nickase nuclease. The mutation of N to A at 902 position amino acid will result in nickase nuclease. The mutation of H to A at 1010 position amino acid will result in nickase nuclease. The mutation of D to A at 1013 position amino acid will result in nickase nuclease.

The simultaneous mutation of E to A at 796 position amino acid and D to A at 1013 position amino acid will result in a Cas9 nuclease that is non-cleaving but retains binding (i.e., dead Cas9).

The helper proteins comprise a Cas1 helper protein, a Cas2 helper protein and a Csn2 helper protein;
wherein the Cas1 helper protein has an amino acid sequence as shown in SEQ ID NO: 2, or an amino acid sequence with at least 80%, 85%, 90%, 95%, 98%, or 99% homology to the amino acid sequence as shown in SEQ ID NO: 2;
wherein the Cas2 helper protein has an amino acid sequence as shown in SEQ ID NO: 3, or an amino acid sequence with at least 80%, 85%, 90%, 95%, 98%, or 99% homology to the amino acid sequence as shown in SEQ ID NO: 3;
wherein the Csn2 helper protein has an amino acid sequence as shown in SEQ ID NO: 4, or an amino acid sequence with at least 80%, 85%, 90%, 95%, 98%, or 99% homology to the amino acid sequence as shown in SEQ ID NO: 4.

The CRISPR RNA is generated by transcription of a CRISPR Array, and has an RNA sequence as shown in SEQ ID NO: 5, or an RNA sequence with at least 80%, 85%, 90%, 95%, 98%, or 99% homology to the nucleic acid sequence as shown in SEQ ID NO: 5; wherein the CRISPR Array comprises a direct repeat sequence and a spacer sequence, wherein the direct repeat sequence has a nucleic acid sequence as shown in SEQ ID NO: 6, or a nucleic acid sequence with at least 80%, 85%, 90%, 95%, 98%, or 99% homology to the nucleic acid sequence as shown in SEQ ID NO: 6; and wherein the spacer sequence has a nucleic acid sequence as shown in SEQ ID NO: 7, or a nucleic acid sequence with at least 80%, 85%, 90%, 95%, 98%, or 99% homology to the nucleic acid sequence as shown in SEQ ID NO: 7.

The trans-activated CRISPR RNA comprises a sequence complementary to a direct repeat sequence of the CRISPR RNA, and the trans-activated CRISPR RNA has a nucleic acid sequence as shown in SEQ ID NO: 8, or a nucleic acid sequence with at least 70%, 80%, 85%, 90%, 95%, 98%, or 99% to the nucleic acid sequence as shown in SEQ ID NO: 8.

The guide RNA formed by hybridizing the crRNA with the tracrRNA has a scaffold composed of a sequence of 7 to 24 nts of a crRNA direct repeat sequence and a tracrRNA sequence; preferably the two parts are fused by a "GAAA", "TGAA", or "AAAC" linker to form an sgRNA scaffold; preferably, an sgRNA scaffold formed by fusion of the crRNA direct repeat sequence of 20 nts and a full length sequence of the tracrRNA by "GAAA" is as shown in SEQ ID NO: 9, and on this basis, preferably, a highly efficient variant of an sgRNA scaffold is an sgRNA scaffold formed by fusion of the first 18 to 14 nts of the crRNA direct repeat sequence and the last 69 to 65 nts of the tracrRNA by a linker sequence (for example, "GAAA"), preferably selected from the following five scaffolds:
(1) an sgRNA scaffold with a length of 91 nts, which comprises 18 nts direct repeat sequence and 69 nts tracrRNA, as shown in SEQ ID NO: 10;
(2) an sgRNA scaffold with a length of 89 nts, which comprises 17 nts direct repeat sequence and 68 nts tracrRNA, as shown in SEQ ID NO: 11;
(3) an sgRNA scaffold with a length of 87 nts, which comprises 16 nts direct repeat sequence and 67 nts tracrRNA, as shown in SEQ ID NO: 12;
(4) an sgRNA scaffold with a length of 85 nts, which comprises 15 nts direct repeat sequence and 66 nts tracrRNA, as shown in SEQ ID NO: 13;
(5) an sgRNA scaffold with a length of 83 nts, which comprises 14 nts direct repeat sequence and 65 nts tracrRNA, as shown in SEQ ID NO: 14;
optionally, the sgRNA scaffold has a nucleic acid sequence with at least 70%, 80%, 85%, 90%, 95%, 98%, or 99% homology to any of SEQ ID NOs: 9 to 14.

The Type II CRISPR/Cas9 genome editing system, derived from *Faecalibaculum rodentium,* binds or cleaves a specific DNA in a biological process (such as genome editing process), by complementary pairing recognition of the guide RNA and a target of the specific DNA, and wherein a length of a paired binding part of the guide RNA and the target of the specific DNA ranges from 14 to 30 bps (preferably 20 to 23 bps); wherein the specific DNA is a DNA of prokaryote or eukaryote.

The length of the paired binding part of the guide RNA and the target of the specific DNA is 21 bps, 22 bps or 23 bps, and wherein the RNP complex is highly sensitive to base mismatch of 14 bps close to a protospacer adjacent motif and the 14 bps is a seed region.

The protospacer adjacent motif required for a function of binding or cleaving DNA is 5'-NNTA-3' downstream of the guide RNA/sgRNA recognition sequence

The disclosure also provides the abovementioned Type II CRISPR/Cas9 genome editing system for use in editing DNA, CRISPR activation or CRISPR interference.

Preferably, the DNA is prokaryotic or eukaryotic DNA. Preferably, the Type II CRISPR/Cas9 genome editing system is derived from *Faecalibaculum rodentium.*

As another aspect of the disclosure, the disclosure further provides the Type II CRISPR/Cas9 genome editing system above for use in the preparation of a nickase, a dead Cas9, a base editor, or a prime editor. Preferably, the preparation is in a prokaryote or a eukaryote.

Compared with the prior art, the disclosure has the beneficial effects as follows.
(1) According to the disclosure, through bioinformatics analysis, a Type II CRSIPR/Cas9 genome editing system in *Faecalibaculum rodentium* is found, and the genome editing system is applied to editing prokaryotic or eukaryotic genes and provides a new option in the genome editing toolbox.
(2) The disclosure provides a Type II CRISPR/Cas9 genome editing system derived from *Faecalibaculum rodentium,* which has new physical and chemical properties and can identify a plurality of different PAM sequences, wherein the specific sequence of PAM recognized by the genome editing system is 5'-NNTA-3' downstream of the guide RNA/sgRNA recognition sequence.
(3) The disclosure provides a Type II CRISPR/Cas9 genome editing system derived from *Faecalibaculum rodentium,* which has high cleavage efficiency on the same DNA site compared with the most common SpCas9, while the off-target is lower than the most common SpCas9, such that it is a safer and more effective genome editing tool.
(4) The disclosure provides a Type II CRISPR/Cas9 genome editing system derived from *Faecalibaculum rodentium,* wherein the PAM recognized at the genome editing system level has palindrome characteristics, and targets therefore are distributed "back-to-back" on a genome, which has a higher density than SpCas9.
(5) The disclosure provides a Type II CRISPR/Cas9 genome editing system derived from *Faecalibaculum rodentium,* which has high flexibility and can be modified into a base editor and a prime editor, and is a genome editing tool capable of being widely applied to different scenarios.

### BRIEF DESCRIPTION OF THE DRAWINGS

Figure 1 is a composition diagram of a Type II CRISPR/Cas9 genome editing system of *Faecalibaculum rodentium* according to the disclosure.
Figure 2 is a structural diagram of Cas9 protein of the Type II CRISPR/Cas9 genome editing system of *Faecalibaculum rodentium* according to the disclosure.

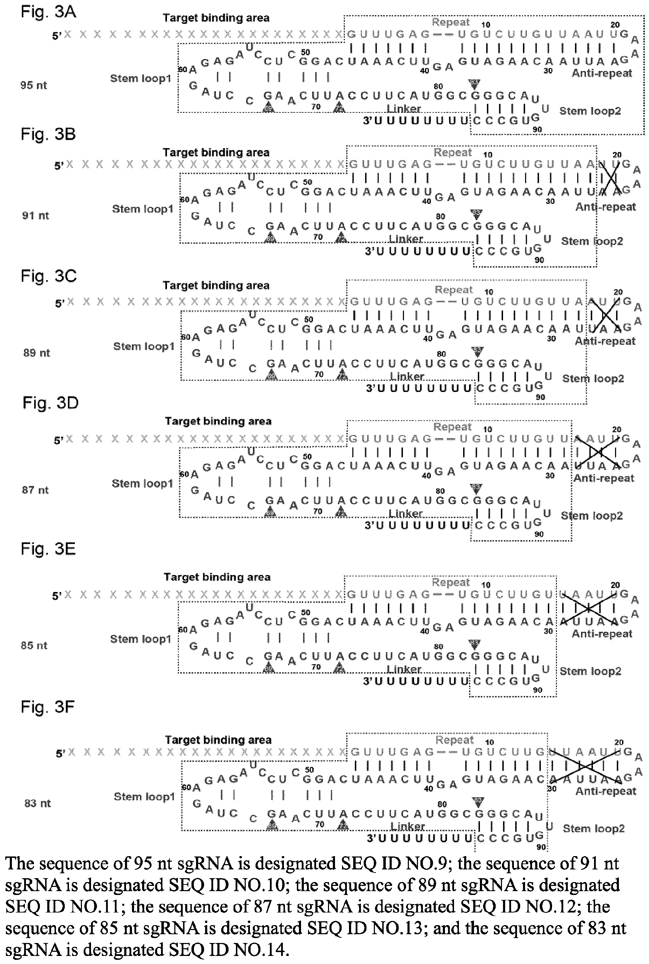

Figure 3 is an RNA secondary structure prediction diagram and an optimal sgRNA scaffold diagram of a guide RNA molecule recognized by the Type II CRISPR/Cas9 genome editing system of *Faecalibaculum rodentium* according to the disclosure. Figure 3A shows the full length 95 nts gRNA. Figure 3B shows an sgRNA scaffold with a length of 91 nts, which comprises 18 nts direct repeat sequence and 69 nts tracrRNA. Figure 3C shows an sgRNA scaffold with a length of 89 nts, which comprises 17 nts direct repeat sequence and 68 nts tracrRNA. Figure 3D shows an sgRNA scaffold with a length of 87 nts, which comprises 16 nts direct repeat sequence and 67 nts tracrRNA. Figure 3E shows an sgRNA scaffold with a length of 85 nts, which comprises 15 nts direct repeat sequence and 66 nts tracrRNA. Figure 3F shows an sgRNA scaffold with a length of 83 nts, which comprises 14 nts direct repeat sequence and 65 nts tracrRNA.
Figure 4 is a schematic diagram of prokaryotic PAM sequences of the Type II CRISPR/Cas9 genome editing system of *Faecalibaculum rodentium* according to the disclosure.
Figure 5 is a schematic diagram of prokaryotic interference experiments of the Type II CRISPR/Cas9 genome editing system of *Faecalibaculum rodentium* according to the disclosure.
Figure 6 is a schematic diagram of eukaryotic cleaving of the Type II CRISPR/Cas9 genome editing system of *Faecalibaculum rodentium* according to the disclosure. Figure 6A is an ODN-PCR gel diagram. Figure 6B is a Sanger sequence peak.
Figure 7 is a schematic diagram showing the optimal length of sgRNA in the Type II CRISPR/Cas9 genome editing system of *Faecalibaculum rodentium* according to the disclosure. Figure 7A is a graph showing the cleavage efficiency of different sgRNA lengths at the HEK293 SITE2-T2 site. Figure 7B is a graph showing the cleavage efficiency of different sgRNA lengths at the DNMT1-T3 site. Figure 7C is a graph showing the cleavage efficiencies of different sgRNA lengths at the RNF2-T6 site.
Figure 8 is a schematic diagram showing the optimal length of the sgRNA recognition sequence of the Type II CRISPR/Cas9 genome editing system of *Faecalibaculum rodentium* according to the disclosure.
Figure 9 is a schematic diagram showing the comparison of the target efficiency and off-target of GUIDE-seq in the Type II CRISPR/Cas9 genome editing system of *Faecalibaculum rodentium* according to the disclosure as compared with SpCas9.
Figure 10 is a graph showing experimental results of the FrCas9-BE base editor; wherein, Figure 10A is a schematic diagram and results of confirming the form of FrCas9 nickase by ODN breakpoint PCR; Figure 10B is the editing window for the nFrCas9-BE4Gram; Figure 10C is the editing window for nFrCas9-ABE7.10; Figure 10D is a Venn diagram of pathogenic mutations in the ClinVar database that can be uniquely corrected by the SpCas9 and FrCas9 base editors; Figure 10E shows the C > T editing efficiency for FrCas9-BE4Gam using 2 "back-to-back" sgRNA simultaneously.
Figure 11 is a diagram showing the distribution of FrCas9 and SpCas9 targets in the human genome, where Figure 11A shows the distribution of 5'-GG-3' (representing SpCas9 PAM) in the GRCh38 human genome; Figure 11B shows the distribution of 5'-TA-3' (representing FrCas9 PAM) in the GRCh38 human genome.
Figure 12 shows the application of FrCas9-specific targeted TATA box in CRISPR interference and CRISPR activation, wherein Figure 12A is a schematic diagram of the TATA-box of the ABCA1 gene targeted by FrCas9; Figure 12B shows CRISPR interference of FrCas9 and SpCas9 by targeting TATA-box, and CRISPR activation of FrCas9 and SpCas9 by targeting TATA-box; Figure 12C shows CRISPR activation of FrCas9 and SpCas9 by targeting the TATA-box.
Figure 13 is a schematic diagram of the Prime Editing genome editing system.
Figure 14 shows the genome editing effect of the FrCas9-PE Prime Editing system verified by Sanger sequencing, wherein the measured sequence was CGAACACTCAAGGTAAT (SEQ ID NO: 33).

### DETAILED DESCRIPTION OF THE DISCLOSURE

In order to better explain the objects, technical solutions and advantages of the disclosure, the disclosure will be further described below with reference to specific embodiments.

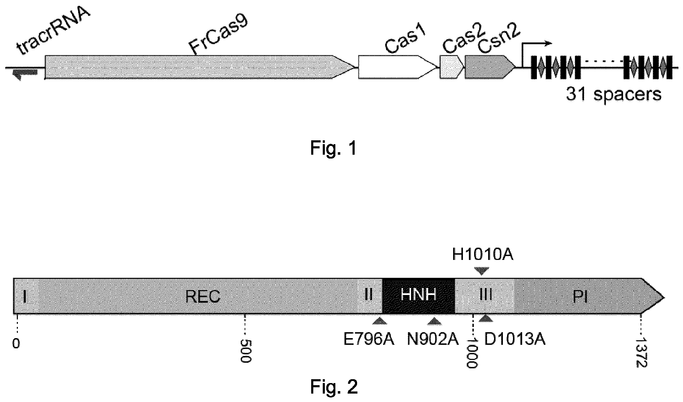

A Type II CRISPR/Cas9 genome editing system derived from *Faecalibaculum rodentium* comprises a Cas9 protein, helper proteins, a CRISPR RNA and a trans-activated CRISPR RNA, as shown in Figure 1. The Cas9 protein is a DNA endonuclease, and the Cas9 protein has an amino acid sequence as shown in SEQ ID NO: 1, or an amino acid sequence with at least 80%, 85%, 90%, 95%, 98%, or 99% homology to the amino acid sequence as shown in SEQ ID NO: 1.

As a preferred embodiment of the Type II CRISPR/Cas9 genome editing system derived from *Faecalibaculum rodentium* according to the disclosure, the Cas9 protein cleaves a double-stranded DNA complementary to crRNA upstream of the PAM sequence by a nuclease domain, as shown in Figure 2. The nuclease domain is selected from a HNH-like nuclease domain, a RuvC-like nuclease domain, or a combination thereof.

The Cas9 protein *(Faecalibaculum rodentium* Cas9), abbreviated as FrCas9 protein, comprises 1372 amino acids (SEQ ID NO: 1), and is a multi-domain and multifunctional DNA endonuclease. It efficiently cleaves a double-stranded DNA complementary to sgRNA upstream of the PAM by a nuclease domain, for example, cleaving a DNA strand complementary to the sgRNA sequence by a HNH-like nuclease domain, or cleaving a non-complementary strand DNA by a RuvC-like nuclease domain. Among them, The mutation of E to A at 796 position amino acid will result in nickase nuclease. The mutation of N to A at 902 position amino acid will result in nickase nuclease. The mutation of H to A at 1010 position amino acid will result in nickase nuclease. The mutation of D to A at 1013 position amino acid will result in nickase nuclease. The simultaneous mutation of E to A at 796 position amino acid and D to A at 1013 position amino acid will result in a Cas9 nuclease that is non-cleaving but retains binding.

As a preferred embodiment of the Type II CRISPR/Cas9 genome editing system derived from *Faecalibaculum rodentium* according to the disclosure, the helper proteins comprise a Cas1 helper protein, a Cas2 helper protein and a Csn2 helper protein. The Cas 1 helper protein has an amino acid sequence as shown in SEQ ID NO: 2, or an amino acid sequence with at least 80%, 85%, 90%, 95%, 98%, or 99% homology to the amino acid sequence as shown in SEQ ID NO: 2. The Cas2 helper protein has an amino acid sequence as shown in SEQ ID NO: 3, or an amino acid sequence with at least 80%, 85%, 90%, 95%, 98%, or 99% homology to the amino acid sequence as shown in SEQ ID NO: 3. The Csn2 helper protein has an amino acid sequence as shown in SEQ ID NO: 4, or an amino acid sequence with at least 80%, 85%, 90%, 95%, 98%, or 99% homology to the amino acid sequence as shown in SEQ ID NO: 4.

The helper proteins Cas1, Cas2 and Csn2 according to the disclosure participate in exogenous gene capture and maturation of crRNA.

As a preferred embodiment of the Type II CRISPR/Cas9 genome editing system derived from *Faecalibaculum rodentium* according to the disclosure, the CRISPR RNA is generated by transcription of a CRISPR Array. The CRISPR RNA has an RNA sequence as shown in SEQ ID NO: 5, or an RNA sequence with at least 80%, 85%, 90%, 95%, 98%, or 99% homology to the nucleic acid sequence as shown in SEQ ID NO: 5. The CRISPR Array comprises a direct repeat sequence and a spacer sequence. The direct repeat sequence is as shown in SEQ ID NO: 6. The spacer sequence is as shown in SEQ ID NO: 7.

The CRISPR RNA(crRNA) according to the disclosure guides the Cas protein to recognize an intruding foreign genome in a base complementary form. When bacteria are exposed to invasion by bacteriophage or virus, a short segment of foreign DNA is integrated as a new spacer between CRISPR repeated spacer sequences in the host chromosome, thereby providing a genetic record of infection. When the body is invaded by a foreign gene again, the CRISPR array transcribes and produces a precursor crRNA(pre-crRNA) with a spacer sequence at the 5' end and a length of 30 bps, which is complementary to the sequence from the foreign invasion gene. The 3' end is a repeat sequence with a length of 36 bps.

As a preferred embodiment of the Type II CRISPR/Cas9 genome editing system derived from *Faecalibaculum rodentium* according to the disclosure, the trans-activated CRISPR RNA comprises a sequence complementary to a direct repeat sequence of the CRISPR RNA as shown in SEQ ID NO: 8.

The trans-activated crRNA(tracrRNA) according to the disclosure is a non-protein encoded RNA, and participates in the maturation of crRNA and the formation of sgRNA. Under the action of the tracrRNA and Cas9 nuclease, the pre-crRNA removes 0 to 16 nts upstream of the spacer sequence and 12 to 29 nts downstream of the repeat sequence to form mature crRNA, and binds the tracrRNA to form a tracrRNA-crRNA complex, which comprises a part recognizing the foreign DNA sequence and has length ranging from 14 to 30 bps. Tetraloop (for example, a "GAAA", "TGAA" or "AAAC" sequence) of four bases may be added between downstream of the crRNA and upstream of the tracrRNA to bind the tracrRNA and the crRNA, in order to form an sgRNA comprising two bulge and three duplex structures upstream, as well as a stem loop structure downstream, which can be further divided into a part that recognizes a foreign DNA sequence and a scaffold part.

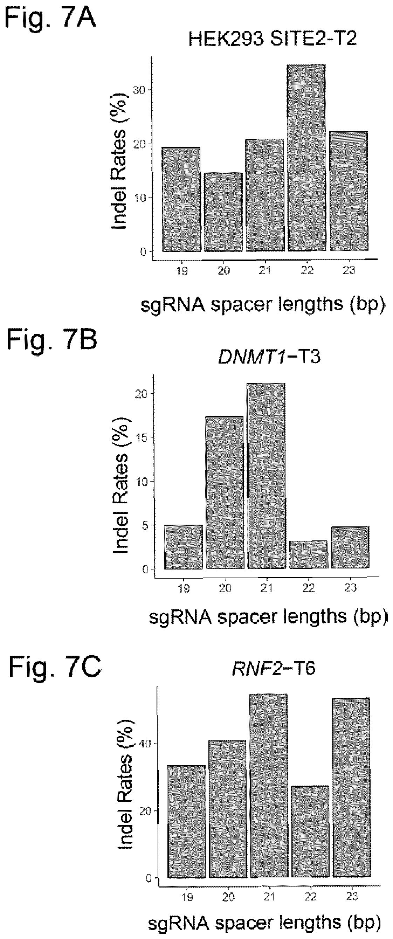

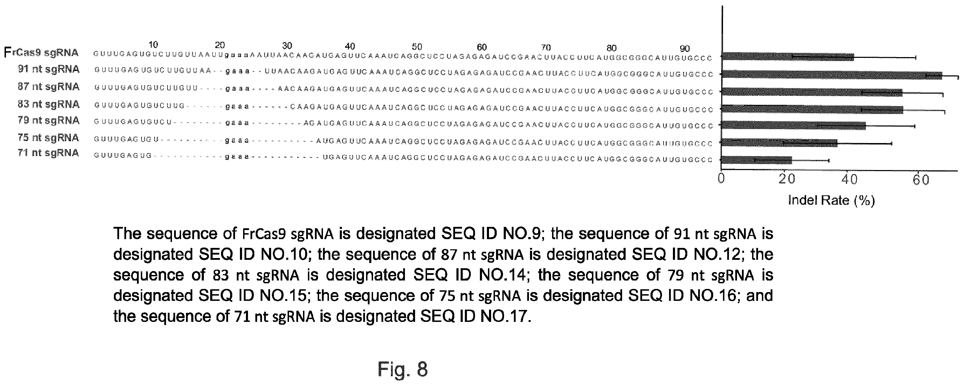

The cleavage by the endonuclease can be further optimized by adjusting the length of the part of the sgRNA recognizing the foreign DNA sequence and the length of the tracrRNA. The early experiments of the disclosure prove that, the optimal length of the part of sgRNA recognizing the exogenous DNA sequence is 21 bps, 22 bps or 23 bps (as shown in Figure 8), and the optimal length of the scaffold part is as follow 5 kinds, as shown in Figures 3 and 7: 91 nts (18 nts crRNA direct repeat + 69 nts tracrRNA), 89 nts (17 nts crRNA direct repeat + 68 nts tracrRNA), 87 nts (16 nts crRNA direct repeat + 67 nts tracrRNA), 85 nts (15 nts crRNA direct repeat + 66 nts tracrRNA), and 83 nts (14 nts crRNA direct repeat + 65 nts tracrRNA).

As a preferred embodiment of the Type II CRISPR/Cas9 genome editing system derived from *Faecalibaculum rodentium* according to the disclosure, the *Faecalibaculum rodentium-derived* Type II CRISPR/Cas9 genome editing system binds or cleaves structures of DNA functions in a genome editing process.

As a preferred embodiment of the Type II CRISPR/Cas9 genome editing system derived from *Faecalibaculum rodentium* according to the disclosure, the DNA is a DNA of prokaryote or eukaryote.

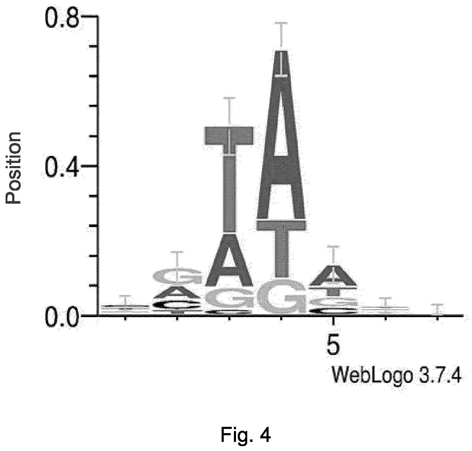

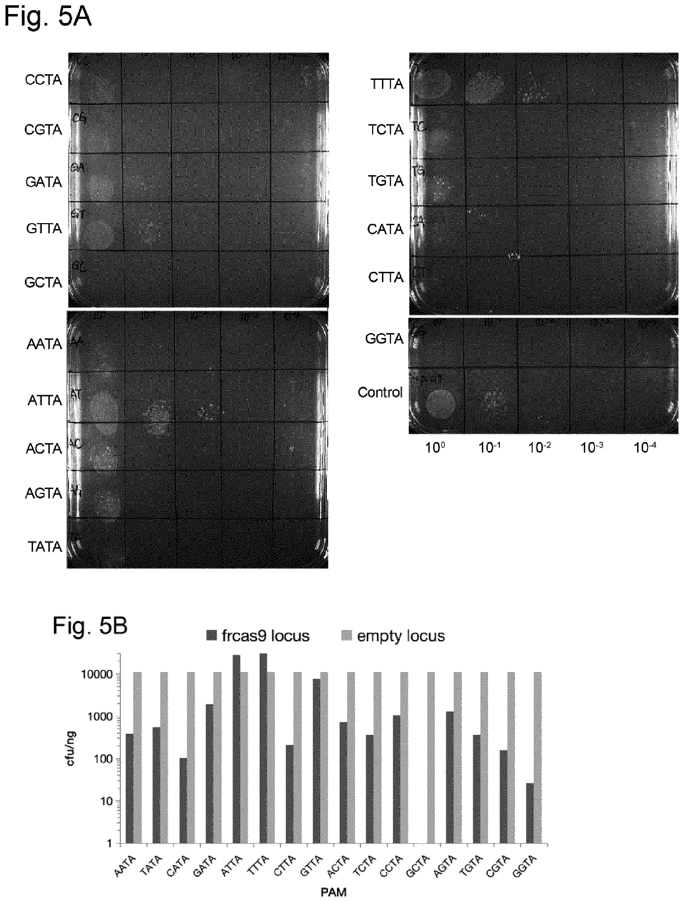

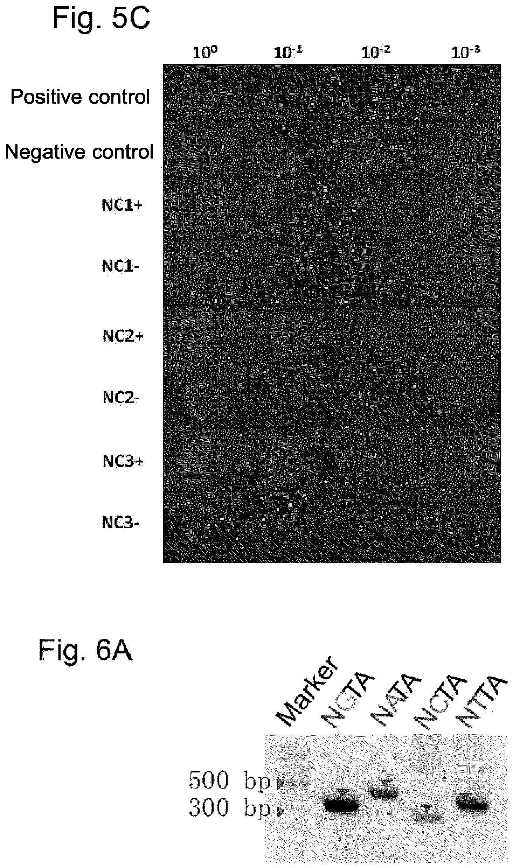

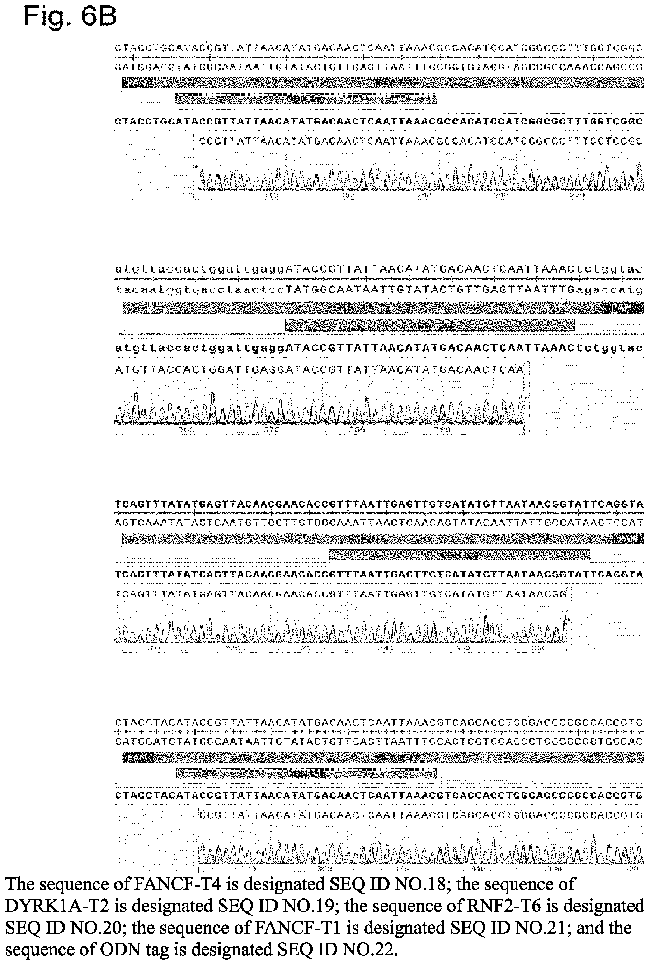

In some embodiments, the FrCas9 according to the disclosure can recognize a variety of DNA double-stranded fragment (DSB) formed by ProSpacer Adjacent Module (PAM) immediately downstream of the targeting sequence. Two important factors are needed for FrCas9 protein to recognize the targeting sequence: one is the nucleotide complementary to crRNA spacer; and the other is the Protospacer Adjacent Motif, PAM) sequence adjacent to the complementary sequence. The depletion experiment shows that FrCas9 has a cleavage effect in the prokaryotic system, and preliminarily verifies that the third and fourth positions of the PAM sequence recognized by this newly discovered Type II CRISPR/Cas9 system are TA, as shown in Figure 4. It is further confirmed by interference experiment that the PAM downstream of the targeting sequence recognized by FrCas9 is 5'-NNTA-3', as shown in Figure 5. All the above PAMs are verified by eukaryotic experiment, as shown in Figure 6. By artificially designing the spacer sequence in the crRNA, this CRISPR-Cas9 system can target almost all DNA sequences of interest in the genome, producing a site-specific flat-ended double-strand break (DSB). Repair of the DSB by non-homologous termini resulting in small random insertions/deletions (indels) at the cleavage site to inactivate the gene of interest. Alternatively, by high-fidelity homologous repair, precise genomic modifications at the DSB site can be performed using homologous repair templates.

Most human genetic diseases are single base mutation, which cannot be treated by traditional methods. The base editor is one of the latest and most effective ways to achieve accurate genome editing. It is characterized by the use of CRISPR-Cas protein in nicking form to locate specific DNA targets, and the use of DNA deaminase to modify and mutate this DNA target to correct the diseased bases without producing double-stranded DNA fragmentation. In some embodiments, the combination of E796A nFrCas9 with the optimized fourth generation cytidine base editor BE4Gam can successfully construct an E796A nFrCas9-BEGam cytosine base editor (CBE), such that the C:G base pair in DNA can be mutated to T:A. The combination of E796A nFrCas9 with the 7th generation adenine base editor ABE7.10 can successfully construct an E796A nFrCas9-BABE7.10 adenine base editor (ABE), such that the A:T base pair in DNA can be mutated to G:C.

In some embodiments, the specific PAM of FrCas9 of the disclosure is NNTA with a specific target on TATA-BOX (one of the elements constituting the eukaryotic promoter), such that FrCas9 specifically targets TATA-BOX to exert a unique CRISPR interference (CRISPRi)/activation (CRISPRa) effect. Among them, CRISPRi can be achieved by directly targeting the cleavage of TATA-BOX with the active form of FrCas9 and destroying TATA-box, or by directly binding to TATA-box with dFrCas9 (i.e., dead Cas9) without cleavage activity. CRISPRa can be achieved by, but not limited to, dFrCas9-VP64 targeting to the TATA-box site.

Prime Editing (PE) is a brand new accurate genome editing tool. The technology can realize the free replacement of single base and the accurate insertion and deletion of multiple bases, greatly reducing the harmful by-products produced by indels in the process of genome editing and significantly improving the editing accuracy. It is widely considered to be a significant advance in genome editing. In some embodiments, the FrCas9 of the disclosure is fused with a reverse transcriptase, and the corresponding pegRNA can be designed according to its PAM sequence to establish a FrCas9-PE genome editing system. Specifically, two sequences are added to the 3' end sequence of pegRNA. The first sequence is a primer binding site (PBS), which can be complementary to the end of a fractured target DNA chain to initiate a reverse transcription process, and the second sequence is a reverse transcription template (RT template), which carries a target point mutation or insertion deletion mutation to achieve accurate genome editing.

A further purpose of the disclosure is to provide the use of the Type II CRISPR/Cas9 genome editing system derived from *Faecalibaculum rodentium* in editing prokaryotic or eukaryotic genes, CRISPR activation or CRISPR interference.

As a preferred embodiment of the use described according to the disclosure, the Type II CRISPR/Cas9 genome editing system derived from *Faecalibaculum rodentium* is used to bind or cleave structures of DNA function at the DNA level.

### Example 1

In the example, the RNA secondary structure of the guide RNA molecule recognized by the Type II CRISPR/Cas9 genome editing system of the *Faecalibaculum rodentium* according to the disclosure was predicted, and the RNA structure after the transcription of the tracrRNA and repeat was predicted by simulating the RNA binding process of the two, and the obtained RNA secondary structure is shown in Figure 3.
(1) Materials: Predicted tracrRNA and repeat sequences, and predicted anti-repeat sequences.
(2) Software: NUPACK (http://www.nupack.org/partition/new)
(3) Prediction method: The in vitro interaction process of 1µl each of two RNAs at 37 °C was simulated by online application of NUPACK, and the secondary structure of the obtained RNA composition was predicted to obtain an RNA secondary structure as shown in Figure 2.

As shown in Figure 3, pre-crRNA removed 0 to 16 nts upstream of the spacer sequence and 12 to 29 nts downstream of the repeat sequence under the action of the tracrRNA and Cas9 nuclease to form mature crRNA which was fused with tracrRNA to form a tracrRNA-crRNA complex containing an exogenous DNA sequence complementary to the spacer sequence, and the sequence length was 14 to 30 bps. They were bound to form sgRNA by the addition of a tetraloop of four bases between the downstream of crRNA and the upstream of the tracrRNA, containing two bulge and three duplex structures upstream and three stem loop structures downstream.

### Example 2

In the example, the Protospacer Adjacent Motif (PAM) recognized by the Type II CRISPR/Cas9 genome editing system of *Faecalibaculum rodentium* in the prokaryotic system was 5'-NNTA-3'.
(1) Materials: Genes related to the CRISPR/Cas9 genome editing system predicted by the above implementation.
(2) Verification method: In this example, a prokaryotic verification system was constructed for the Type II CRISPR/Cas9 genome editing system of *Faecalibaculum rodentium* according to the disclosure, to verify its cleavage effect and preliminarily explore the recognized PAM sequence by a second generation sequencing technology, and the result is shown in Figure 4.

Detailed protocols are as follows.
(a) The *Faecalibaculum rodentium* Type II CRISPR/Cas9 genome editing system (comprising an endonuclease Cas9, helper proteins Cas1, Cas2, Csn2, a CRISPR array and a non-coding RNA tracrRNA) of the disclosure was inserted into a pACYC184 vector, wherein the Cas9 protein was subjected to *escherichia coli* codon optimization. The natural spacer sequences and spacer sequences in library were added into the CRISPR array, and a strong heterologous promoter J23119 was added on the Cas9 protein and the CRISPR array to construct a prokaryotic expression plasmid of pACYC184-FrCas9.
(b) Seven random bases (16,384 insertions in total) were added into the spacer sequence 3' of library. Two restriction endonuclease sites of EcoRI and NcoI were selected from MCS polyclonal of pUC19 vector. The library was cloned into the vector and a target-library plasmid was constructed.
(c) The plasmid containing pACYC184-FrCas9 or empty pACYC184 and target-library were co-electrically transfected to E. coli DH5a. After resuscitation at 25 °C for 2h, they were uniformly spread on SOB medium containing double resistance of ampicillin sodium (100 µ g/mL) and chloramphenicol (34ug/ml) for incubation at 25 °C for 30h, and the plasmid was collected by alkaline lysis.
(d) PCR amplification on a region containing a spacer sequence and seven random bases was performed. The secondary sequencing was performed on two ends of a PCR product by adding a linker. The PAM depletion value (PPDV) relative to a no-load control group was calculated. The PAM sequence of the Type II CRISPR/Cas9 genome editing system of the *Faecalibaculum rodentium* was generated by using Weblogo, wherein the PAM sequence is 5'-NNTA-3'.

Figure 3 is a schematic diagram of a conservative PAM sequence recognized in a prokaryotic system by the Type II CRISPR/Cas9 genome editing system of *Faecalibaculum rodentium* according to the disclosure. The second-generation sequencing analysis was performed on library DNA obtained through an depletion experiment to calculate the PAM depletion value (PPDV) relative to a no-load control group. The PAM sequence of FrCas9 generated by Weblogo is 5'-NNTA-3'.

### Example 3

In the example, the Protospacer Adjacent Motif (PAM) recognized in the Type II CRISPR/Cas9 genome editing system of *Faecalibaculum rodentium* according to the disclosure was verified by an interference experiment, and its cleaving ability at the prokaryotic level and potential genome editing ability in eukaryotes were determined. The results of interference experiments are shown in Figure 5, and the schematic diagrams of various possible PAM sequences recognized by the Type II CRISPR/Cas9 genome editing system of *Faecalibaculum rodentium* according to the disclosure are shown in Figure 5.
(1) Materials: The pACYC184-FrCas9, target-library plasmid obtained in Example 4, and the preliminarily recognized PAM.
(2) Verification method: In the example, the Protospacer Adjacent Motif (PAM) recognized in a prokaryotic system by the Type II CRISPR/Cas9 genome editing system of the *Faecalibaculum rodentium* according to the disclosure was further determined through an interference experiment.

Detailed protocols are as follows.
(a) A total of 16 combined sequences were obtained by adding NNTA to the 3' position of the spacer sequence, and the target plasmid was constructed by cloning into pUC19 through the restriction endonuclease sites of EcoRI and NcoI, respectively.
(b) The 16 target plasmids were respectively transfected into E. coli DH5a electrogenic competence containing FrCas9-related loci, and the plasmids were gradually diluted after resuscitation at 25 °C for 2 h. The target plasmids were incubated overnight at 25 °C in SOB medium containing double resistance of ampicillin sodium (100ug/ml) and chloramphenicol (34ug/ml) by dot blot, to observe the number of monoclonal bacteria.

Figure 5 is a schematic diagram of an interference experiment of the Type II CRISPR/Cas9 genome editing system of *Faecalibaculum rodentium* according to the disclosure. Based on the NNTA found in the depletion experiment, 16 target plasmids of different combinations of NNs were constructed. The monoclonal colony count was observed through the interference experiment. In Figure 5A, the leftmost column was designated as the single FrCas9, and the right side was designated as the target plasmid targeted by FrCas9 cleavage. The results showed that the colony count in the right column was decreased. Figure 5B is a statistical diagram of the cleaving effects of a plurality of PAM sequences recognized by the Type II CRISPR/Cas9 genome editing system of *Faecalibaculum rodentium* according to the disclosure, and the Figure illustrates that cfu of target plasmids of 14 different combinations of NN are decreased as compared with that of the control group. The above results indicated that the Protospacer Adjacent Motif (PAM) recognized by the newly discovered CRISPR/Cas9 system in the prokaryotic system was 5'-NNTA-3' (N represents any base selected from the group consisting of A, T, C and G) by interference experiment.

### Example 4

In the example, the tracrRNA range required for cleavage of targeted DNA sequences in the Type II CRISPR/Cas9 genome editing system of *Faecalibaculum rodentium* according to the disclosure was verified by interference experiments, and the results of the interference experiments are shown in Figure 5C.
(1) Materials: pACYC184-FrCas9, target plasmid, preliminarily recognized PAM obtained in Example 5.
(2) Verification method: In the example, the tracrRNA range required for cleaving the targeted DNA sequence was further determined in a prokaryotic system by the Type II CRISPR/Cas9 genome editing system of *Faecalibaculum rodentium.*

Detailed protocols are as follows.
(a) By a Gibson clone method, a total of six gene sequence of all possible non-coding regions in wild-type *Faecalibaculum rodentium* were cloned into a target plasmid to construct target-NC 1 to 6 plasmids, wherein NC6 is formed by splice the full length of the non-coding regions, and NCs 1 to 5 respectively represent the possible non-coding regions of the first to fifth segments. In each NC plasmid, a strong heterologous promoter J23119 was added upstream of the non-coding region, wherein "+" represented a forward non-coding region and "-" represented a reverse non-coding region, respectively;
(b) From the pACYC184-FrCas9 obtained in Example 5, all possible non-coding regions were removed by a PCR homologous recombination method, and the CRISPR related protein and the CRISPR array gene sequence were retained, thus constructing a pACYC184-ΔFrCas9;
(c) The 12 target plasmids were respectively transfected into E.coli DH5a electrogenic competence containing pACYC184-ΔFrCas9, and the plasmids were gradually diluted after resuscitation at 25 °C for 2 h. The target plasmids were incubated overnight at 25 °C in SOB medium containing double resistance of ampicillin sodium (100ug/ml) and chloramphenicol (34ug/ml) by dot blot, to observe the number of monoclonal bacteria.

Figure 5C is a schematic diagram of the interference experiment of the Type II CRISPR/Cas9 genome editing system of *Faecalibaculum rodentium* according to the disclosure, and the results show that the monoclonal colonies of NCI and NC6 were significantly less than those of NCs 2 to 5, indicating that the alternative non-coding region in the first segment can assist FrCas9 in effectively and targeted cleavage of DNA sequences in E. coli.

### Example 5

In the example, the ability to cleave targeted DNA sequences in eukaryotic cells was verified by an ODN experiment in the Type II CRISPR/Cas9 genome editing system of *Faecalibaculum rodentium.* An ODN-PCR result is shown in a diagram in Figure 6A, and a Sanger sequencing result is shown in a diagram in Figure 6B.
(1) Materials: All amino acid sequences, CRISPR array sequences, tracrRNA sequences, and the recognized PAM of the *Faecalibaculum rodentium* editing gene obtained in Examples 1-6.
(2) Verification method: In the example, the ability to cleave targeted DNA sequences in eukaryotic cells was verified through an ODN experiment in the Type II CRISPR/Cas9 genome editing system of *Faecalibaculum rodentium.*

Detailed protocols are as follows.
(a) The human-derived optimized FrCas9 protein sequence was synthesized and cloned into a PX330 eukaryotic vector to construct a PX330-FrCas9 plasmid.
(b) 30 bps upstream of NGTA, NATA, NCTA and NTTA on a human RNF2 gene were selected as a target sequence. A CRISPR array was constructed by a Gibson method. A human eukaryotic strong promoter U6 was added upstream of the CRISPR array, to construct a PX330-FrCas9-array plasmid.
(c) A mouse-derived eukaryotic strong promoter U6 was added upstream of the tracrRNA determined in Example 4 to construct a PX330-FrCas9-array-tracrRNA plasmid.
(d) The PX330-FrCas9-array-tracrRNA plasmid targeting different gene sites constructed above with 2.5ug and 1.5ul ODN was electrically transfected into HEK293T cells in good condition. All the cells were collected after 72 h for extraction of DNA.
(e) The ODN-PCR was performed by designing a pair of primers near the *RNF2* targeted gene site and on the ODN sequence, and agarose gel electrophoresis was performed to observe whether there was a band for preliminary identification of the occurrence of targeted cleavage event.
(f) Sanger sequencing verified the successful insertion of ODN into the target site, and confirmed that FrCas9 had the ability to edit the target DNA in eukaryotic cells.

The results of ODN-PCR are shown in Figure 6A. NGTA, NATA, NCTA and NTTA all had target bands with correct band sizes. As shown in Figure 8 of Sanger sequencing results, the insertion position of ODN occurred in the 3 to 4 bps base upstream of PAM, indicating that the sequence cleavage range of target gene in FrCas9 was consistent with that in the previous SpCas9.

### Example 6

In the example, the optimal length of the sgRNA recognition part in eukaryotic cells in the Type II CRISPR/Cas9 genome editing system of *Faecalibaculum rodentium* according to the disclosure was verified by an ODN experiment, and the results are shown in Figure 7.
(1) Materials: All amino acid sequences, sgRNA sequences and recognized PAM; of the *Faecalibaculum rodentium* editing gene obtained in Examples 1-6;
(2) Verification method: In the example, the optimal length of the sgRNA recognition part in the eukaryotic cells in the Type II CRISPR/Cas9 genome editing system of the *Faecalibaculum rodentium* was verified through an ODN experiment.

Detailed protocols are as follows.
(a) The human-derived optimized FrCas9 protein sequence was synthesized and cloned into a PX330 eukaryotic vector to construct a PX330-FrCas9 plasmid.
(b) A target sequence of 30bp upstream of GGTA near the target site of SpCas9 which was found to have a good cleavage effect in the previous study was selected as the target sequence, and an sgRNA with a recognition length of 19 to 23 bps was constructed by Gibson method. A human eukaryotic strong promoter U6 was added upstream of the sgRNA to construct a PX330-FrCas9-sgRNA plasmid.
(c) The PX330-FrCas9-sgRNA plasmid targeting different gene sites constructed above with 2.5ug and 1.5ul ODN was electrically transfected into HEK293T cells in good condition. All the cells were collected after 72 h for extraction of DNA.
(d) The ODN-PCR was performed by designing a pair of primers near the target gene site and on the ODN sequence, and agarose gel electrophoresis was conducted to observe whether there was a band for preliminary identification of targeted cleavage event and to compare the band strength of sgRNA with the recognition length of 19 to 23 bps.
(e) Amplicon high-throughput database was established to quantify the Indel rate of the target region and the cleavage effects of sgRNA with a recognition length of 19 to 23 bps were compared.

As shown in Figure 7, the optimal sgRNA recognition length for FrCas9 was 21 bps, 22 bps, or 23 bps.

### Example 7

In the example, the optimal length of an sgRNA scaffold in eukaryotic cells in the Type II CRISPR/Cas9 genome editing system of *Faecalibaculum rodentium* according to the disclosure was verified by an ODN experiment, and the results are shown in Figure 8.
(1) Materials: All amino acid sequences, sgRNA sequences and recognized PAM; of the *Faecalibaculum rodentium* editing gene obtained in Examples 1-6.
(2) Verification method: in the example, the ODN experiment was used for verifying the optimal length of an sgRNA scaffold of the eukaryotic cells in the Type II CRISPR/Cas9 genome editing system of the *Faecalibaculum rodentium.*

Detailed protocols are as follows.
(a) The human-derived optimized FrCas9 protein sequence was synthesized and cloned into a PX330 eukaryotic vector to construct a PX330-FrCas9 plasmid.
(b) A target sequence of 30bp upstream of GGTA near the target site of SpCas9 which was found to have a good cleavage effect in the previous study was selected as the target sequence, and an sgRNA with a scaffold length of 71 nts to 95 nts was constructed by Gibson method. A human eukaryotic strong promoter U6 was added upstream of the sgRNA to construct a PX330-FrCas9-sgRNA plasmid.
(c) The PX330-FrCas9-sgRNA plasmid targeting different gene sites constructed above with 2.5ug and 1.5ul ODN was electrically transfected into HEK293T cells in good condition. All the cells were collected after 72 h for extraction of DNA.
(d) The ODN-PCR was performed by designing a pair of primers near the targeted gene site and on the ODN sequence, and agarose gel electrophoresis was conducted to observe the presence of bands to preliminarily identify whether targeted cleavage occurred or not, and the band strengths of sgRNA with the scaffold length of 71 nts to 95 nts were compared.
(e) Amplicon high-throughput database was established to quantify the Indel rate of the target region and the cleavage effects of sgRNA with a scaffold length of 71 nts to 95 nts were compared.

As shown in Figures 8 and 3, the optimal sgRNA scaffold for FrCas9 is 83 nt to 91 nts.

### Example 8

In the example, the cleaving effect in eukaryotic cells in the Type II CRISPR/Cas9 genome editing system of *Faecalibaculum rodentium* as higher than that of SpCas9 and the off-target rate as lower than that of SpCas9 was verified by an ODN experiment.
(1) Materials: All amino acid sequences, 22 bps sgRNA sequence, 5'-GGTA-3' PAM sequence, SpCas9 amino acid sequence, SpCas9 sgRNA sequence and SpCas9 5'-NGG-3' PAM sequence of the *Faecalibaculum rodentium* editing gene obtained in Examples 1-6;
(2) Verification method: In the example, the GUIDE-seq experiment was used to verify that the cleaving effect in eukaryotic cell nuclear cells of the Type II CRISPR/Cas9 genome editing system of *Faecalibaculum rodentium* according to the disclosure was higher than that of SpCas9 and the off-target rate was lower than that of SpCas9.

Detailed protocols are as follows.
(a) The human-derived optimized FrCas9 protein sequence was synthesized and cloned into a PX330 eukaryotic vector to construct a PX330-FrCas9 plasmid.
(b) A target sequence of 30bp upstream of GGTA near the target site of SpCas9 which was found to have a good cleavage effect in the previous study was selected as the target sequence, and an sgRNA with a recognized length of 22 bp was constructed by Gibson method. A human eukaryotic strong promoter U6 was added upstream of the sgRNA to construct a PX330-FrCas9-sgRNA plasmid, and at the same time, construct a PX330-SpCas9-sgRNA plasmid of 20 bps SpCas9 sgRNA.
(c) The PX330-FrCas9-sgRNA plasmid and PX330-SpCas9-sgRNA plasmid targeting different gene sites constructed above with 2.5ug and 1.5ul ODN was electrically transfected into HEK293T cells in good condition. All the cells were collected after 72 h for extraction of DNA.
(d) The ODN-PCR was performed by designing a pair of primers near the targeted gene site and on the ODN sequence, and agarose gel electrophoresis was conducted to observe the presence of bands to preliminarily identify whether targeted cleavage occurred or not, and the DNA with bands was subjected to GUIDE-seq database building.
(e) Through bioinformatics analysis, the target-cleaving effects and off-targets of SpCas9 and FrCas9 at the same site were compared.

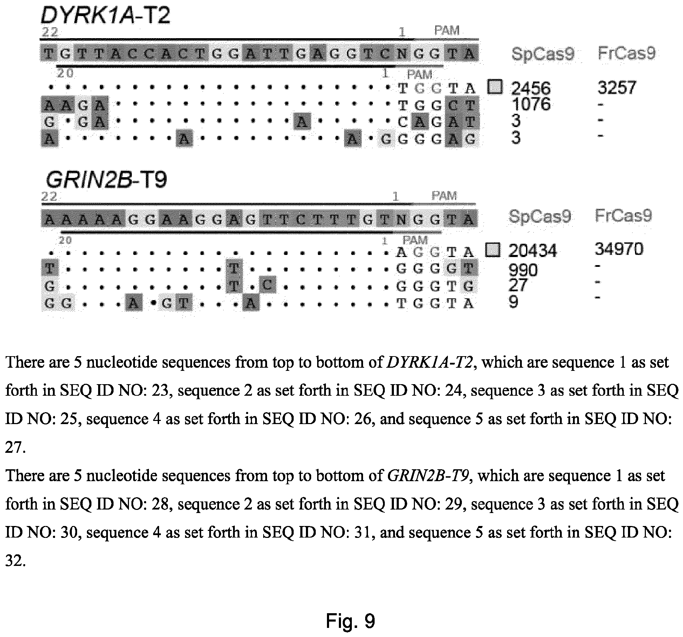

As shown in Figure 9, FrCas9 was located at target Reads digit 3257 at DYRK1A-T2 site, higher than that of SpCas9 at target Reads 2456. Meanwhile, off-target was not detected for FrCas9, while SpCas9 showed off-target at three sites. FrCas9 was at target Reads digit 34970 at the GRIB2B-T9 site, which was higher than that of SpCas9 at target Reads 20434. Meanwhile, off-target was not detected for FrCas9, while SpCas9 showed off-target at 3 sites. The above data indicated that FrCas9 was a CaS9 protein superior to SpCas9 in cleavage efficiency and specificity.

### Example 9 FrCas9 can be used for Prime Editing (PE)

Most human genetic diseases are single base mutation, which cannot be treated by traditional methods. The base editor is one of the latest and most effective ways to achieve accurate genome editing. It is characterized by the use of CRISPR-Cas protein in nicking form to locate specific DNA targets, and the use of DNA deaminase to modify and mutate this DNA target to correct the diseased bases without producing double-stranded DNA fragmentation. The cytosine base editor (CBE) mutated the C:G base pair in DNA to T:A, and the adenine base editor (ABE) mutated the A:T base pair to G: C.

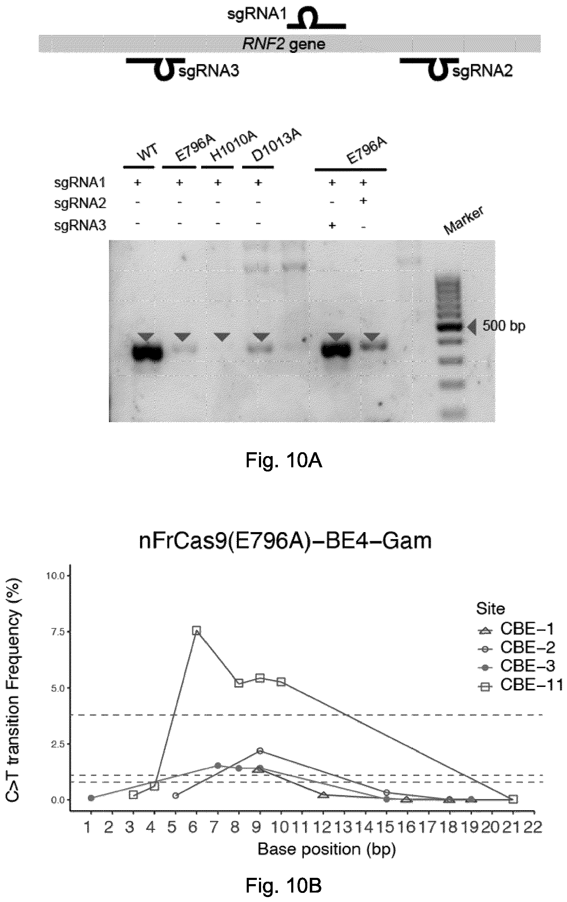

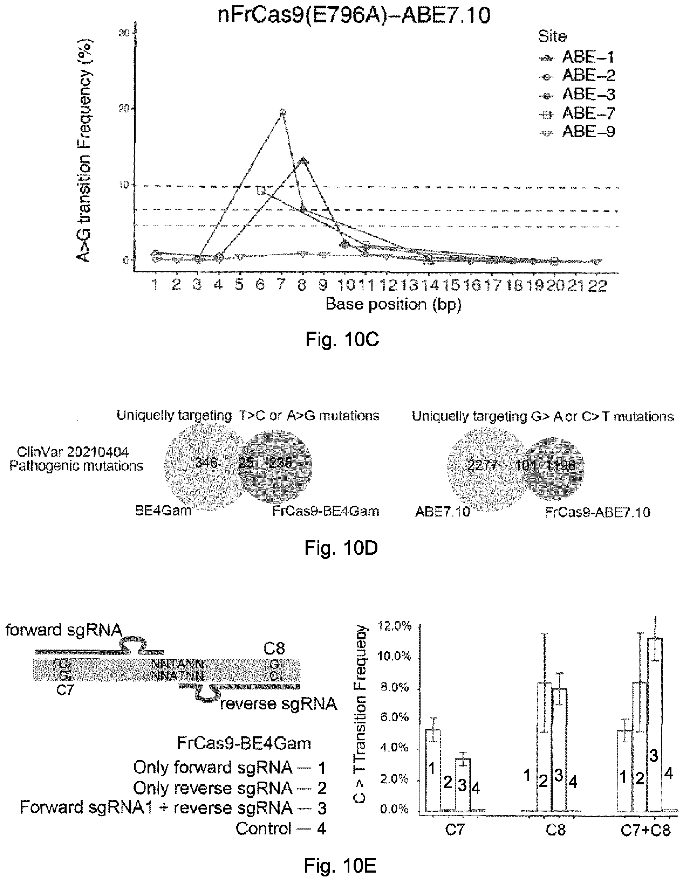

First, three point-mutations of E796A, H1010A and D1013A were respectively incorporated to generate different FrCas9 nickases (nFrCas9). Then, E796A nFrCas9 with the optimized fourth-generation cytidine base editor BE4Gam and seventh-generation adenine base editor ABE7.10 were combined. It is observed that the editing window of FrCas9-BE4Gam was 6^{th}-10^{th} (Figure 10B) bases and that of FrCas9-ABE7.10 was 6^{th}-8^{th} bases (Figure 10C). Based on the above characteristics, the targeting scopes of FrCas9-BE4Gam and FrCas9-ABE7.10 in ClinVar databases were calculated. For pathogenic mutations that could be precisely corrected by FrCas9-BE4Gam, 90.38% (235/260) unique events were different from SpCas9-BE4Gam. For pathogenic mutations that could be precisely corrected by FrCas9-ABE7.10, 92.21% (1196/1297) unique events were different from SpCas9-ABE7.10 (Figure 10D). Therefore, the TA-rich PAM of FrCas9 greatly expanded the targets in human genome for base-editor to correct human disease-associated mutations.

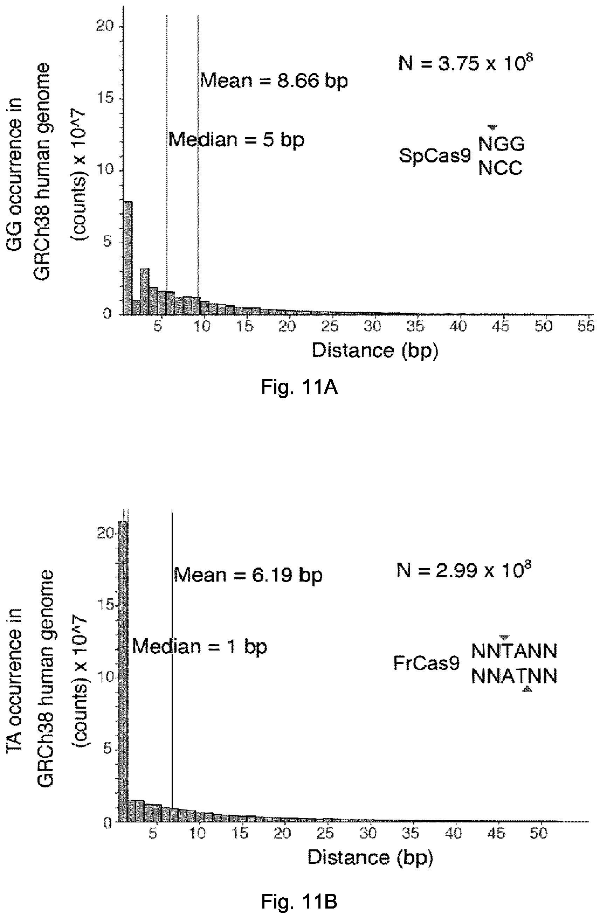

Furthermore, the PAM of FrCas9 (5'-NNTA-3') was palindromic, which offered pairwise "back-to-back" existence of sgRNAs (Figure 10E). This feature could broaden the scopes of FrCas9 base-editors by modifying two close editing windows at the same time (Figure 10E) and increase the target distribution and density of FrCas9 sgRNAs. The 5'-GG-3' (represented for SpCas9 PAM) and 5'-TA-3' (represented for FrCas9 PAM) distributions in human genomes were calculated (Figures 11A and 11B). Compared to SpCas9 (median = 5 bp, mean = 8.66 bp)⁵, FrCas9 showed more intensive distributions (median = 1 bp, mean = 6.16 bp) in human genomes, providing additional applicable loci.

### Example 10 FrCas9 can target TATA-box to modulate gene expression as an effective tool for CRISPR activation and inhibition

TATA-BOX(TATA box/Hogness box) is one of the elements that constitute the promoter of eukaryotes. The consistent order is TATA(A/T)A(A/T) (non-template chain sequence). It is about -30 bp (-25 to-32 bp) upstream of the transcription initiation point of most eukaryotic genes and basically consists of A-T base pairs, which is the selection for determining the transcription initiation of genes. It is one of the binding sites of RNA polymerase, which can only start transcription after firmly binding to TATA-BOX. Since the PAM sequence of FrCas9 is NNTA, it has natural advantages in targeting TATA-BOX.

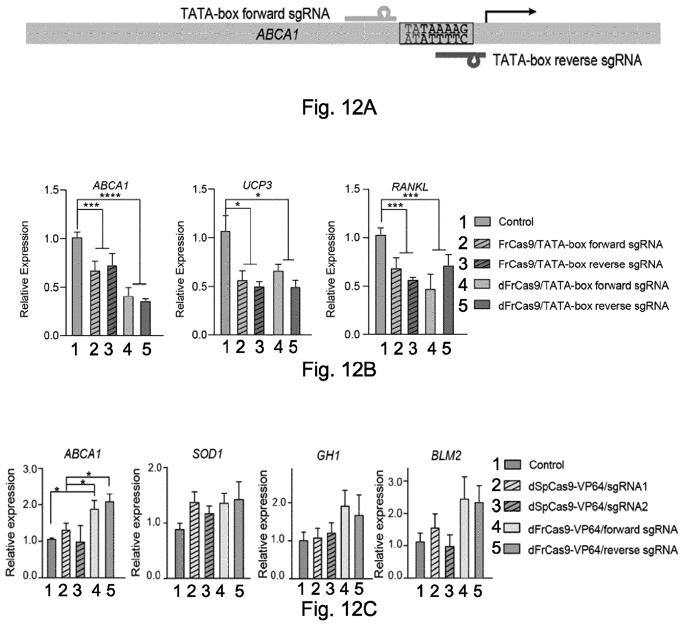

FrCas9 CRISPR interference (CRISPRi) in three TATA-box promoted genes, *ABCA1, UCP3* and *RANKL* were tested (Figure 12A). By cleaving the TATA-box, FrCas9 reduced *ABCA1, UCP3* and *RANKL* expression by 31.37%, 49.91% and 39.62%, respectively. Meanwhile, dFrCas9 without cleavage activity to directly bind to TATA-box was also utilized, and the expression of *ABCA1, UCP3* and *RANKL* decreased 61.67%, 45.61% and 42.60% by dFrCas9 directly binding to the TATA-box, respectively (Figure 12B). Accordingly, FrCas9 possesses unique potential for efficient genome engineering of TATA-box related genetic diseases.

Further, FrCas9 CRISPR activation (CRISPRa) using dFrCas9-VP64 directly targeting the TATA-box was tested, and its performance was compared with dSpCas9-VP64 targeting the upstream of TATA-box. The CRISPRa experiments were conducted in *ABCA1, SOD1, GH1* and *BLM2* genes. The results showed that dFrCas9-VP64 enables effective transcriptional activation. Moreover, the fold activation of dFrCas9-VP64 in *ABCA1, GH1* and *BLM2* was higher than that of dSpCas9-VP64, while the fold activation of *SOD1* gene was comparable to that of dSpCas9-VP64 (Figure 12C). Therefore, FrCas9 is a promising tool for CRISPR screening due to its unique 5'-NNTA-3' PAM.

### Example 11 FrCas9 can be used for Prime Editing, PE

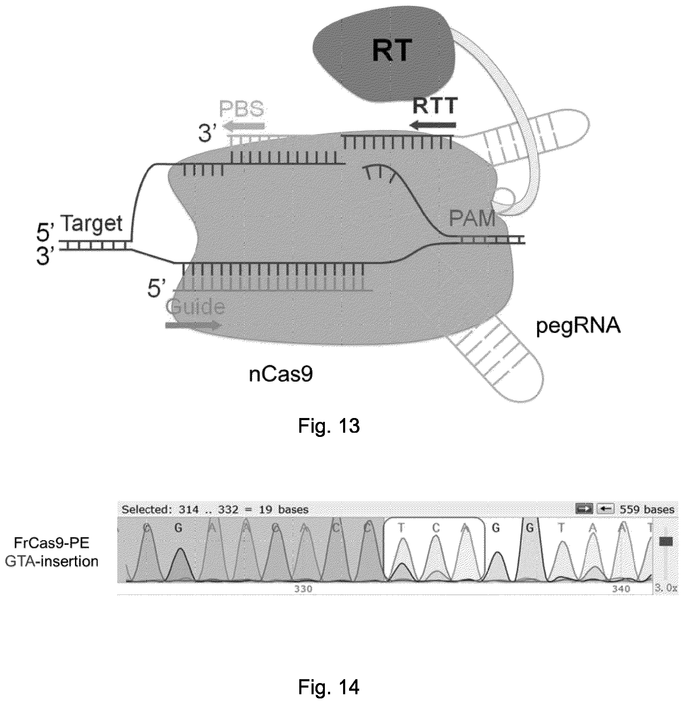

Prime Editing (PE) is a brand-new precise gene editing tool. This technology can greatly reduce the harmful by-products produced by indels during the gene editing process, significantly improve the editing accuracy, and has the potential to overcome fundamental barriers to the treatment of genetic diseases with existing gene editing methods. The PE system consists of two components, which comprises an engineered guide RNA (pegRNA) and a prime editor protein. pegRNAs have dual functions: the capable of directing the edited protein to the target site and containing the edited template sequence. The prime editor protein consists of a mutated Cas9 nickase (which cuts only one DNA strand) and a reverse transcriptase fused. After Cas9 cleaves the target site, reverse transcriptase uses the pegRNA as a template for reverse transcription, thereby incorporating the desired edit into the DNA strand, and the corrected sequence preferentially replaces the original genomic DNA, thereby permanently editing the target site (Figure 13).

Based on the biological characteristics of FrCas9, FrCas9 with reverse transcriptase was fused, and the corresponding pegRNA was designed according to its PAM sequence, and the FrCas9-PE gene editing system was established, and its gene editing efficiency was optimized. The pegRNA was designed according to the FrCas9 PAM sequence. Compared with sgRNA, the 3'-end sequence of pegRNA has two additional sequences. The first sequence is the primer binding site (PBS), which can be complementary to the end of the broken target DNA strand to initiate reverse transcription. In the process, the second sequence is a reverse transcription template (RT template), which carries target point mutations or indel mutations to achieve precise gene editing. Previous studies have shown that the length of the PBS and RT template sequences will significantly affect the gene editing efficiency of the PE system, and it varies by gene locus. Therefore, we explored the optimization of editing efficiency through the combination of PBS and RT template sequences of different lengths.

At the cellular level, the gene editing effect of the FrCas9-PE system was preliminarily verified. In HEK293T cells, the HEK293T-RNF2 gene locus, which is commonly used for CRISPR gene editing efficiency evaluation, was targeted to verify the gene editing function of the FrCas9-PE system. Our current experimental results show that FrCas9-PE can produce site-specific base editing effects. After that, we will further transform the existing FrCas9-PE, including codon optimization, nuclear localization sequence position and number optimization, reverse transcriptase modification, etc (Figure 14

## Claims

1. A Type II CRISPR/Cas9 genome editing system, **characterized in that**, the system comprises a Cas9 protein, helper proteins, a crRNA(CRISPR RNA) and a tracrRNA (trans-activated CRISPR RNA) in the functional form of an RNP complex (ribonucleoprotein complex) of the Cas9 protein and a guide RNA formed by hybridizing the crRNA with the tracrRNA;
wherein the Cas9 protein is a DNA endonuclease, and the Cas9 protein has an amino acid sequence as shown in SEQ ID NO: 1, or an amino acid sequence with at least 80%, 85%, 90%, 95%, 98%, or 99% homology to the amino acid sequence as shown in SEQ ID NO: 1.

2. The Type II CRISPR/Cas9 genome editing system according to claim 1, **characterized in that**, the Cas9 protein cleaves a double-stranded DNA complementary to a crRNA upstream of a PAM sequence by a nuclease domain, wherein the nuclease domain is selected from a HNH-like nuclease domain, a RuvC-like nuclease domain, or a combination thereof.

3. The Type II CRISPR/Cas9 genome editing system according to claim 1, **characterized in that**, the helper proteins comprise a Cas1 helper protein, a Cas2 helper protein and a Csn2 helper protein;
wherein the Cas1 helper protein has an amino acid sequence as shown in SEQ ID NO: 2, or an amino acid sequence with at least 80%, 85%, 90%, 95%, 98%, or 99% homology to the amino acid sequence as shown in SEQ ID NO: 2;
wherein the Cas2 helper protein has an amino acid sequence as shown in SEQ ID NO: 3, or an amino acid sequence with at least 80%, 85%, 90%, 95%, 98%, or 99% homology to the amino acid sequence as shown in SEQ ID NO: 3;
wherein the Csn2 helper protein has an amino acid sequence as shown in SEQ ID NO: 4, or an amino acid sequence with at least 80%, 85%, 90%, 95%, 98%, or 99% homology to the amino acid sequence as shown in SEQ ID NO: 4.

4. The Type II CRISPR/Cas9 genome editing system according to claim 1, **characterized in that**, the CRISPR RNA is generated by transcription of a CRISPR Array, and has an RNA sequence as shown in SEQ ID NO: 5, or an RNA sequence with at least 80%, 85%, 90%, 95%, 98%, or 99% homology to the nucleic acid sequence as shown in SEQ ID NO: 5; wherein the CRISPR Array comprises a direct repeat sequence and a spacer sequence, wherein the direct repeat sequence has a nucleic acid sequence as shown in SEQ ID NO: 6, or a nucleic acid sequence with at least 80%, 85%, 90%, 95%, 98%, or 99% homology to the nucleic acid sequence as shown in SEQ ID NO: 6; and wherein the spacer sequence has a nucleic acid sequence as shown in SEQ ID NO: 7, or a nucleic acid sequence with at least 80%, 85%, 90%, 95%, 98%, or 99% homology to the nucleic acid sequence as shown in SEQ ID NO: 7.

5. The Type II CRISPR/Cas9 genome editing system according to claim 1, **characterized in that**, the trans-activated CRISPR RNA comprises a sequence complementary to a direct repeat sequence of the CRISPR RNA, and the trans-activated CRISPR RNA has a nucleic acid sequence as shown in SEQ ID NO: 8, or a nucleic acid sequence with at least 70%, 80%, 85%, 90%, 95%, 98%, or 99% to the nucleic acid sequence as shown in SEQ ID NO: 8.

6. The Type II CRISPR/Cas9 genome editing system according to claim 1, **characterized in that**, the guide RNA formed by hybridizing the crRNA with the tracrRNA has a scaffold composed of a sequence of 7 to 24 nts of a crRNA direct repeat sequence and a tracrRNA sequence; preferably the two parts are fused by a "GAAA", "TGAA", or "AAAC" linker to form an sgRNA scaffold;
preferably, an sgRNA scaffold formed by fusion of the crRNA direct repeat sequence of 20 nts and a full length sequence of the tracrRNA by "GAAA" is as shown in SEQ ID NO: 9, and on this basis, preferably, a highly efficient variant of an sgRNA scaffold is
an sgRNA scaffold formed by fusion of the first 18 to 14 nts of the crRNA direct repeat sequence and the last 69 to 65 nts of the tracrRNA by a linker sequence (for example, "GAAA"), preferably selected from the following five scaffolds:
(1) an sgRNA scaffold with a length of 91 nts, which comprises 18 nts direct repeat sequence and 69 nts tracrRNA, as shown in SEQ ID NO: 10;
(2) an sgRNA scaffold with a length of 89 nts, which comprises 17 nts direct repeat sequence and 68 nts tracrRNA, as shown in SEQ ID NO: 11;
(3) an sgRNA scaffold with a length of 87 nts, which comprises 16 nts direct repeat sequence and 67 nts tracrRNA, as shown in SEQ ID NO: 12;
(4) an sgRNA scaffold with a length of 85 nts, which comprises 15 nts direct repeat sequence and 66 nts tracrRNA, as shown in SEQ ID NO: 13;
(5) an sgRNA scaffold with a length of 83 nts, which comprises 14 nts direct repeat sequence and 65 nts tracrRNA, as shown in SEQ ID NO: 14;
optionally, the sgRNA scaffold has a nucleic acid sequence with at least 70%, 80%, 85%, 90%, 95%, 98%, or 99% homology to any of SEQ ID NOs: 9 to 14.

7. The Type II CRISPR/Cas9 genome editing system according to claim 1, **characterized in that**, the Type II CRISPR/Cas9 genome editing system is derived from *Faecalibaculum rodentium,* and wherein a specific DNA is bound or cleaved in a genome editing process, by complementary pairing recognition of the guide RNA and a target of the specific DNA, and wherein a length of a paired binding part of the guide RNA and the target of the specific DNA ranges from 14 to 30 bps;
wherein the specific DNA is a DNA of prokaryote or eukaryote.

8. The Type II CRISPR/Cas9 genome editing system according to claim 7, **characterized in that**, the length of the paired binding part is 21 bps, 22 bps or 23 bps, and wherein the RNP complex is highly sensitive to base mismatch of 14 bps close to a protospacer adjacent motif and the 14 bps is a seed region.

9. The Type II CRISPR/Cas9 genome editing system according to claim 7, wherein a protospacer adjacent motif required for a function of binding or cleaving DNA is 5'-NNTA-3' downstream of the guide RNA/sgRNA recognition sequence, wherein N is A, T, C or G.

10. The Type II CRISPR/Cas9 genome editing system according to any one of claims 1 to 9 for use in editing or binding DNA, CRISPR activation, or CRISPR interference.

11. The Type II CRISPR/Cas9 genome editing system according to claim 10, wherein the DNA is prokaryotic or eukaryotic DNA.

12. The Type II CRISPR/Cas9 genome editing system according to any one of claims 1 to 9 for use in the preparation of a nickase, a dead Cas9, a base editor, or a prime editor.

13. The Type II CRISPR/Cas9 genome editing system according to claim 12, wherein the preparation is in a prokaryote or a eukaryote.
